# EUROPEAN PATENT SPECIFICATION

(11) **EP 0 576 691 B1**
(45) Date of publication and mention of the grant of the patent: **21.01.1998**
(21) Application number: 92109177.3
(22) Date of filing: 01.06.1992
(51) Int. Cl.: C11D 1/66

(54) **Detergent composition**
Detergenszusammensetzung
Composition détergente

(43) Date of publication of application: 05.01.1994
(73) Proprietor: LION CORPORATION, Sumida-ku Tokyo (JP)
(72) Inventor: Ishikawa, Toshiyuki, Sumida-ku, Tokyo (JP); Nishida, Shigeo, Sumida-ku, Tokyo (JP); Ukiya, Chisato, Sumida-ku, Tokyo (JP); Kanao, Hirofumi, Sumida-ku, Tokyo (JP)
(74) Representative: Kador & Partner

(56) References cited:
- EP-A- 0 409 005
- EP-A- 0 427 210
- WO 80/00452
- WPI Derwent, Abstract of JP-A-3223398

## Description

### BACKGROUND OF THE INVENTION

### 1. Field of the Invention

The present invention relates to a detergent composition which is superior in sudsing subsidence and smooths and moisturizes the hand and to a liquid detergent composition superior in low temperature stability.

### 2. Description of the Related Art

Since kitchen cleaners, household cleaners, shampoos, body shampoos, clothing detergents, and other detergents inevitably contact the skin of the hand etc. during washing, studies have been made in the past to develop detergent compositions which are mild to the skin and do not give a greasy feeling.

A sugar ester-based surfactant comprised of an ester of a fatty acid having 6 to 18 carbon atoms and a monoalkyl ether of a monosaccharide having 5 to 6 carbon atoms is known as a surfactant which causes little irritation and is mild to the skin. When such sugar ester-based surfactants are formulated into a detergent, however, there is the problem of a deterioration of the sudsing subsidence, in particular the rinse-off property at low temperatures. Further, detergents having such sugar ester-based surfactants formulated therein have not necessarily been sufficient in terms of the moist feeling given to the skin after use. The moisture of the skin is lost and chaffing due to abrasion and drying is felt. Also, when such sugar ester-based surfactants are formulated into a liquid detergent, the low temperature stability becomes worse. Therefore, when the liquid detergent composition is stored at a low temperature in cold regions etc., the problem of nonhomogenity and turbidity arises.

### SUMMARY OF THE INVENTION

Therefore, the object of the present invention is to eliminate the above problems of the prior art and to improve the rinse-off property of a detergent composition in which sugar ester-based surfactants are formulated.

In accordance with the present invention, there is provided a detergent composition comprising:
(i) 0.5 percent to 50 percent by weight, based upon the total weight of the composition, of at least one ester of a C₈, C₁₀ or C₁₂ fatty acid with a methyl or ethyl ether of xylose, glucose or mannose, and
(ii) 0.001 to 10 percent by weight, based upon the total weight of the composition, of at least one compound selected from glucose, xylose, mannose, ethyl glucose, ethyl maltose and maltotriose.

### DESCRIPTION OF THE PREFERRED EMBODIMENTS

The fatty acid may be saturated or unsaturated or straight chain or branched. Specific examples of such a fatty acid are caprylic acid, capric acid, lauric acid, caproleic acid and lauroleic acid.

The esters are preferably a monoester/diester type mixture including a monoester type and diester type where a fatty acid residue having 8, 10 or 12 carbon atoms is ester-bonded to a hydroxy group of the alkyl ether compound, wherein the polyesters of triesters or higher esters are desirably not more than 1 percent by weight.

The component (i) is preferably included in the detergent composition of the present invention in an amount of 1 to 25 percent by weight. When the amount contained is too small, the cleaning effect and the effect of improvement of the greasy feeling are not sufficiently obtained. On the other hand, even if the amount contained is made large, no improvement is seen in the cleaning performance and the stability of the system is also degraded.

The monosaccharide or the oligosaccharide and alkyl ether of the same of the component (ii) of the present invention is formulated into the detergent composition in an amount of 0.001 to 10 percent by weight, preferably 0.1 to 8 percent by weight. When the component (ii) is added in an amount of over 0.001 percent by weight, the skin of the hand is made to feel smooth and moist after washing. On the other hand, when the component (ii) is formulated in an amount of more than 10 percent by weight, the stability of the detergent composition with respect to contamination by microorganisms is deteriorated.

It is possible to suitably add in the liquid detergent composition of the present invention various components depending upon a desired application, for example, anionic surfactants, nonionic surfactants, cationic surfactants, bipolar surfactants, and other surfactants or other additives.

Examples of the anionic surfactants are as follows;
1) Alkyl sulfuric acid salts of 10 to 18 carbon atoms
2) Alkane sulfonic acid salts of 10 to 18 carbon atoms
3) Olefin sulfonic acid salts of 10 to 18 carbon atoms
4) Alkyl benzene sulfonic acid salts of 10 to 18 carbon atoms in the alkyl group
5) Polyoxyethylene alkyl (or alkenyl) ether sulfuric acid salts (average number of added moles of the ethylene oxide of 2 to 7) of 10 to 18 carbon atoms in the alkyl group or alkenyl group
6) Sulfonic acid salts of lower alkyl esters of fatty acids (number of carbon atoms of fatty acid of 10 to 20, number of carbon atoms of alkyl group of 1 to 3)

Examples of such salts are alkaline metal salts, alkaline earth metal salts, ammonium salts, alkanol amine salts, etc.

In addition, it is possible to add polyoxyethylene alkyl ethers, polyoxyethylene·polyoxypropylene alkyl ethers, alkyl amine oxides, alkanol amines, fatty acid alkanol amides, lower alcohols, polyhydric alcohols, lower aryl sulfonic acid salts, and other hydrotropic agents; zeolite and other inorganic builders, ethylenediamine 4-acetic acid salt, diethylene triamine 5-acetic acid salt, and other metal ion sequestering agents; carbonic acid salts, silicic acid salts, and other alkali builders; benzoic acid and other antibacterial agents, protease, lipases, cellulase, amylase, and other enzymes; colorants, fragrances, and the like.

The detergent composition of the present invention can be used as a detergent for various applications, but it is particularly suited to applications where the detergent composition comes into contact with the hand, skin, scalp, etc. and for washing hard surfaces such as of dishes, tile, and glass. For example, it is suitably used for shampoos, body shampoos, kitchen cleaners, bathtub cleaners, toilet cleaners, and other household cleaners, clothing detergents, etc.

By using it, it is possible to retain the moisture of the skin and give a moist feeling to the hand and to prevent chaffing due to abrasion and drying.

### Example

The present invention will now be further illustrated by, but is by no means limited to, the following example.

The liquid detergents of the compositions shown in Table 1 and Table 2 were prepared and the moist feeling of the hand was evaluated. Note that the indication of "C₈ methyl glucose ester" etc. in the tables means "ester of fatty acid having 8 carbon atoms and methyl glucose" etc.

### Method of evaluation of Moistness of hand

The hand was immersed for 5 minutes in a 10 percent by weight aqueous solution of the composition, then was rinsed with running water and dried with a towel. The moist feeling at that time was evaluated by the senses based on the following standards. The scores used were the average values of a 5 member panel. Here, a moist feeling means the retention of moisture of the skin and no feeling of chaffing due to abrasion or drying.

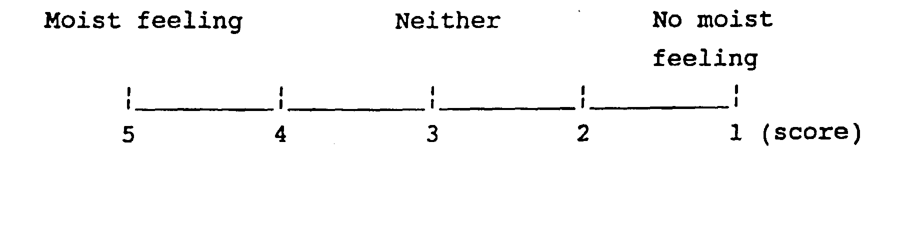

**Table 1**

| Composition | No. of sample (wt% below) | | | | |
|---|---|---|---|---|---|
| | 1 | 2 | 3 | 4 | 5 |
| **Component (a)** | | | | | |
| C₈ methyl glucose ester | 10 | 10 | - | - | 10 |
| C₁₀ ethyl xylose ester | - | - | 10 | - | - |
| C₁₂ methyl mannose ester | - | - | - | 10 | - |

| **Component (b)** | | | | | |
|---|---|---|---|---|---|
| Glucose | 0.1 | - | - | - | - |
| Xylose | - | - | 2 | - | - |
| Mannose | - | - | - | 5 | - |
| Methyl glucose | - | 1 | - | - | - |
| Maltotriose | - | - | - | - | 5 |

| **Optional components** | | | | | |
|---|---|---|---|---|---|
| Sodium C₁₁, C₁₃ alkyl sulfate | 5 | 5 | - | - | 5 |
| Sodium C₁₂, C₁₃ alkyl ether sulfate | - | - | 5 | - | - |
| Sodium C₁₀ to C₁₈ α-olefin sulfonate | - | - | - | 0.5 | - |
| Higher alcohol polyoxyethylene ether (p=12)*1 | - | - | - | 5 | - |
| Lauryl dimethyl amine oxide | 2 | 2 | 2 | 2 | 2 |
| Coconut fatty acid diethanol amide | 5 | 5 | 5 | 5 | 5 |
| Ethanol | 5 | 5 | 5 | 5 | 5 |
| Sodium benzoate | 2 | 2 | 2 | 2 | 2 |
| Sodium toluene sulfonate | 2 | 2 | 2 | 2 | 2 |
| Water | Bal | Bal | Bal | Bal | Bal |
| Moist feeling of hand (average score) | 4.4 | 4.6 | 4.6 | 4.8 | 4.8* |
| 1) p indicates the average added moles of ethylene oxide. | | | | | |

**Table 2**

| Composition | No. of sample of Ex.3 (wt% below) | | | | |
|---|---|---|---|---|---|
| | 6 | 7 | 8 | 9 | 10 |
| **Component (a)** | | | | | |
| C₈ methyl glucose ester | 15 | 15 | - | 10 | 10 |
| C₁₀ ethyl xylose ester | - | - | 15 | - | - |
| C₁₂ methyl mannose ester | - | - | - | - | - |

| **Component (b)** | | | | | |
|---|---|---|---|---|---|
| Glucose | 3 | - | - | - | - |
| Xylose | - | - | 7 | - | - |
| Mannose | - | - | - | - | - |
| Lactose | - | - | - | - | 2 |
| Methyl glucose | - | 7 | - | - | - |
| Ethyl maltose | - | - | - | 2 | - |

| **Optional components** | | | | | |
|---|---|---|---|---|---|
| Sodium C₁₁, C₁₃ alkyl sulfate | - | - | - | 5 | - |
| Sodium C₁₂, C₁₃ alkyl ether sulfate | - | - | - | - | - |
| Sodium C₁₀ to C₁₈ α-olefin sulfonate | - | - | - | - | - |
| Higher alcohol polyoxy ethylene ether (p=12) | - | - | - | - | 5 |
| Lauryl dimethyl amine oxide | 2 | 2 | 2 | 2 | 2 |
| Coconut fatty acid diethanol amide | 5 | 5 | 5 | 5 | 5 |
| Ethanol | 5 | 5 | 5 | 5 | 5 |
| Sodium benzoate | 2 | 2 | 2 | 2 | 2 |
| Sodium toluene sulfonate | 2 | 2 | 2 | 2 | 2 |
| Water | Bal | Bal | Bal | Bal | Bal |
| Moist feeling of hand (average score) | 4.8 | 4.8 | 4.8 | 4.5 | 4.6 |

## Claims

1. A detergent composition comprising:
(i) 0.5% to 50% by weight, based upon the total weight of the composition, of at least one ester of a C₈, C₁₀ or C₁₂ fatty acid with a methyl or ethyl ether of xylose, glucose or mannose and
(ii) 0.001% to 10% by weight, based upon the total weight of the composition of at least one compound selected from glucose, xylose, mannose, ethyl glucose, ethyl maltose, and maltotriose.

## Patentansprüche

1. Eine Detergenszusammensetzung, dadurch gekennzeichnet, daß sie aus:
(i) 0,5 bis 50 Gewichtsprozent, basierend auf dem Gesamtgewicht der Zusammensetzung, wenigstens eines Esters einer C₈-, C₁₀- oder C₁₂-Fettsäure mit einem Methyl- oder Ethylether von Xylose, Glucose oder Mannose und aus
(ii) 0,001 bis 10 Gewichtsprozent, basierend auf dem Gesamtgewicht der Zusammensetzung, wenigstens einer Komponente, die aus Glucose, Xylose, Mannose, Ethylglucose, Ethylmaltose und Maltotriose ausgewählt wurde, besteht.

## Revendications

1. Composition détergente comprenant :
(i) 0,5 % à 50 % en poids, par rapport au poids total de la composition, d'au moins un ester d'un acide gras en C₈, C₁₀ ou C₁₂ avec un éther éthylique ou méthylique de xylose, glucose ou mannose et
(ii) 0,001 % à 10 % en poids, par rapport au poids total de la composition, d'au moins un composé choisi parmi le glucose, le xylose. le mannose, l'éthyl glucose, l'éthyl maltose, et le maltotriose.
